# EUROPÄISCHE PATENTSCHRIFT

(11) **EP 0 405 376 B1**
(45) Veröffentlichungstag und Bekanntmachung des Hinweises auf die Patenterteilung: **18.09.1996**
(21) Anmeldenummer: 90111950.3
(22) Anmeldetag: 23.06.1990
(51) Int. Cl.: C12Q 1/70, C12Q 1/68, C07H 21/04, C12P 19/34

(54) **Typisierung humaner Rhinoviren**
Typing of human rhinoviruses
Caractérisation de rhinovirus humains

(30) Priorität: 24.06.1989 DE 3920754
(43) Veröffentlichungstag der Anmeldung: 02.01.1991
(73) Patentinhaber: BOEHRINGER INGELHEIM INTERNATIONAL GmbH, D-55216 Ingelheim am Rhein (DE)
(72) Erfinder: Torgersen, Helge, Dr., A-3002 Purkersdorf (AT); Skern, Timothy, Dr., A-1160 Wien (AT); Blaas, Dieter, Dipl.-Ing. Dr., A-1090 Wien (AT)

(56) Entgegenhaltungen:
- EP-A- 0 229 701
- EP-A- 0 309 969
- WO-A-87/02065
- JOURNAL OF MEDICAL VIROLOGY Bd. 28, Nr. 2, Juni 1989, NEW YORK, USA Seiten 73 - 77; GAMA, R.E. ET AL.: 'amplification of rhinovirus specific nucleic acids from clinical samples using the polymerase chain reaction'
- NUCLEIC ACIDS RESEARCH Bd. 16, Nr. 19, 11. Oktober 1988, OXFORD, UK Seite 9346; GAMA, R.E. ET AL.: 'polymerase chain reaction amplification of rhinovirus nucleic acids from clinical material'
- ARCHIVES OF VIROLOGY Bd. 90, 1986, BERLIN Seiten 165 - 172; MORINET, F ET AL: 'comparison of 17 isolates of the human parvovirus b19 by restriction enzyme analysis'

## Beschreibung

Gegenstand der vorliegenden Erfindung ist ein Verfahren zur Typisierung humaner Rhinoviren. In zusammenhang mit der vorliegenden Erfindung bezieht sich der Begriff Viren immer auf Rhinoviren.

Eine allgemeine Methode zum Nachweis von Viren beschreibt die Europäische Patentanmeldung Nr. 229701. Bestimmte virale Sequenzen wurden mittels spezifischer Primer in einer "Polymerase Chain Reaction" (PCR; Saiki et al., 1988) amplifiziert und die so erhaltenen DNA-Moleküle mittels spezifischer markierter Proben nachgewiesen.

Die "Polymerase Chain Reaction" ermöglicht die enzymatische Amplifizierung auch unbekannter DNA-Sequenzen in vitro. Eingesetzt werden hierzu zwei Oligonukleotid-Primer, die das zu amplifizierende DNA-Fragment flankieren. Diese Primer sind so konstruiert, daß der eine an den (+)-Strang, der andere an den (-)-Strang bindet, und so orientiert, daß die DNA Synthese durch die DNA-Polymerase über die zwischen den Primern liegende Region erfolgt. Durch die mehrfache, vorzugsweise bis zu dreißigfache Wiederholung eines Zyklus von drei Stufen: 1.) Hitzedenaturierung der DNA; 2.) Binden der Primer an die komplementären Sequenzen; 3.) Verlängerung mit DNA-Polymerase (in jedem Zyklus wird die Menge des DNA-Fragmentes verdoppelt), kommt es zu einer exponentiellen Anhäufung des von den Primern flankierten DNA-Fragments. Durch den vorzugsweisen Einsatz einer thermostabilen DNA-Polymerase, beispielsweise aus dem Bakterium Thermus aquaticus kann die PCR automatisiert werden.

Die europäische Patentanmeldung Nr. 309969 und die internationale Patentanmeldung WO 87/02065 beschreiben Verfahren zur Identifizierung und zum Vergleich spezifischer DNA-Sequenzen durch Vergleich der Restriktionsfragmente der zu analysierenden DNA-Sequenzen.

Morinet et al. (1986) beschreiben den Vergleich verschiedener humaner Parvovirus B19-Typen mittels Restriktions-Analyse. Dabei wurden die vollständigen Genome der zu untersuchenden Viren mittels Restriktionsendonukleasen verdaut und die Restriktionsmuster miteinander verglichen. Der Nachweis der Restriktionsfragmente erfolgte mittels Hybridisierung gegen eine radioaktiv markierte Parvovirus-DNA-Probe.

Über 100 Serotypen humaner Rhinoviren (HRV), die Hauptverantwortlichen für Erkältungskrankheiten, sind mittlerweile beschrieben worden (Stott & Killington, 1972; Cooney et al., 1982; Hamparian et al., 1987). Obwohl die durch rhinovirale Infektionen verursachten Krankheiten normalerweise selbst nicht ernsthafter Natur sind, kann es zu Sekundärinfektionen des geschwächten Organismus kommen; diese Sekundärinfektionen sind von ökonomischer und sozialer Bedeutung. Trotz der beachtlichen Fortschritte in dem Verständnis dieser Virusgruppe, ist eine effektive Impfung bisher aufgrund der Anzahl an Serotypen noch immer ausgeschlossen. Die Diagnose eines Rhinovirus sowie die Bestimmung des Serotyps, der innerhalb einer Population zirkuliert, kann zur Zeit nur durch aufwendige serologische Typisierung erreicht werden (Cooney et al., 1982; Kellner et al., 1988).

Bisher sind die Nukleotidsequenzen der RNA-Genome von 4 HRV-Stämmen bestimmt worden: HRV1B (Hughes et al., 1988), HRV2 (Skern et al., 1985), HRV14 (Stanway et al., 1984); Callahan et al., 1985) und HRV89 (Duechler et al. 1987). Eine Analyse der Sequenzen ergab, daß es signifikante Bereiche von Sequenzidentitäten innerhalb der 5'-nicht-kodierenden Regionen dieser und anderer picornaviraler Genome gibt (Rivera et al., 1988). Zwei solcher Blocks (Klammersequenzen) sind in den vier bisher sequenzierten Rhinovirus-Serotypen konserviert.

Diese Blocks weisen einmal 23 identische Nukleotide auf, nämlich zwischen den Nukleotiden # 531 bis 553 (1. Klammersequenz) sowie einmal 21 identische Nukleotide zwischen # 161 bis 181 (2. Klammersequenz). Die Positionsangaben beziehen sich, soweit nicht anders angegeben, auf die Numerierung des HRV2 (Skern et al. 1985).

Gama et al., (1988 u. 1989) beschreiben die Amplifikation des zwischen diesen Klammersequenzen liegenden und diese umfassenden Abschnitts mittels spezifischer Primer durch PCR. Als Primer wurden für den Bereich der 1. Klammersequenz ein Oligonukleotid verwendet, welches komplementär zur antigenomischen "Sense" DNA der HRV14 Nukleotide 182-197 ist. Für den Bereich der 2. Klammersequenz wurde ein Primer verwendet der komplementär zur genomischen "Sense" DNA im Bereich der Nukleotide 547-562 von HRV14 ist. Mittels dieser Methodik wurden Rhinoviren in klinischen Proben gelelektrophoretisch nachgewiesen.

Zwei aus den konservierten Regionen abgeleitete Primer bieten daher die Möglichkeit, sie universell zur Amplifikation auch unbekannter DNA-Sequenzen einzusetzen.

Zur Typisierung von Viren bzw. Serotypen von Viren ist es erforderlich, charakteristische Unterschiede zwischen den einzelnen Vertretern aufzuzeigen.

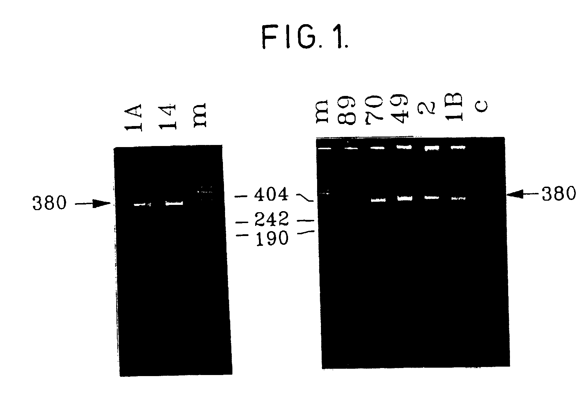

Mit Hilfe der PCR und zweier aus den konservierten Regionen abgeleiteter Primer, insbesondere ein 18- und ein 14-mer, vorzugsweise der Oligonukleotid-Primer 1 (Sequenz # 161 bis 178) und der Oligonukleotid-Primer 2 (komplementär zur Sequenz # 531 bis 544, die nach bekannten Oligonukleotidsyntheseverfahren hergestellt werden können, war es möglich, wie in Figur 1 gezeigt, DNA-Fragmente der Rhinoviren HRV1A, HRV49 und HRV70 zu generieren, neben Fragmenten der von der Sequenz her bekannten Rhinoviren . Die Konservierung der Klammersequenzen erstreckt sich also nicht nur auf die bereits von ihrer Sequenz her aufgeklärten Rhinovirus Serotypen sondern zumindest auch auf drei nicht von der Sequenz her bekannte Serotypen. Die beschriebenen Primer bilden daher die Grundlage der erfindungsgemäßen Typisierungsmethode.

Zur Typisierung von Viren bzw. Serotypen von Viren ist es erforderlich, charakteristische Unterschiede zwischen den einzelnen Vertretern aufzuzeigen. Bisher wurden zur Typisierung, wie bereits erwähnt, die Reaktion mit Antikörpern herangezogen und die Kreuzreaktivitäten bestimmt. Eine andere Möglichkeit ist die Aufdeckung struktureller Unterschiede; eine Methode, die sich normalerweise kaum als schnelles und einfaches diagnostisches Mittel verwenden läßt, ist die Strukturaufklärung in der Regel doch sehr aufwendig. Andererseits ist eine Typisierung aufgrund struktureller Unterscheidungsmerkmale als ausgesprochen sicher anzusehen.

Die Aufgabe der vorliegenden Erfindung bestand daher darin eine vereinfachte Typisierungsmethode für humane Rhinoviren zu entwickeln, die die sich Unterschiede im Bereich zwischen den Klammersequenzen zwischen verschiedenen Viren zunutze macht.

Gelöst wurde diese Aufgabe dadurch, daß man erfindungsgemäß bestimmte "Indikatoren" für strukturelle Unterschiede ausnutzte.

Diese "Indikatoren" können im Falle von DNA bzw. RNA spezielle Restriktionsenzymerkennungsstellen sein. (Die Methode ist insofern allgemein auf Nukleinsäuresequenzen anwendbar als RNA durch reverse Transkription in DNA umgeschrieben werden kann.) Wie dem Fachmann bekannt, sind Restriktionsenzyme im allgemeinen äußerst spezifisch. So kann bereits in vielen Fällen schon die Änderung einer Base innerhalb einer speziellen Erkennungssequenz dazu führen, daß das spezielle Restriktionsenzym die Sequenz nicht mehr erkennt.

Um diese spezifischen "Indikatoren" beispielsweise für die Rhinoviren zu ermitteln, wurden die amplifizierten Fragmente sowohl der strukturell bekannten Rhinovirus-Serotypen als auch der bisher strukturell unbekannten Rhinovirus-Serotypen miteinander verglichen.

Überraschenderweise weisen die vier strukturell bekannten Serotypen trotz des hohen Konservierungsgrades untereinander signifikante Unterschiede zwischen den Regionen identischer Klammersequenzen auf. Die sich hieraus ergebenden charakteristischen Unterschiede im Restriktionsmuster jedes Serotyps, die auch in den von diesen Regionen ausgehenden amplifizierten Fragmenten vorhanden sind, werden erfindungsgemäß verwendet, um die der Erfindung zugrunde liegende Aufgabe zu lösen.

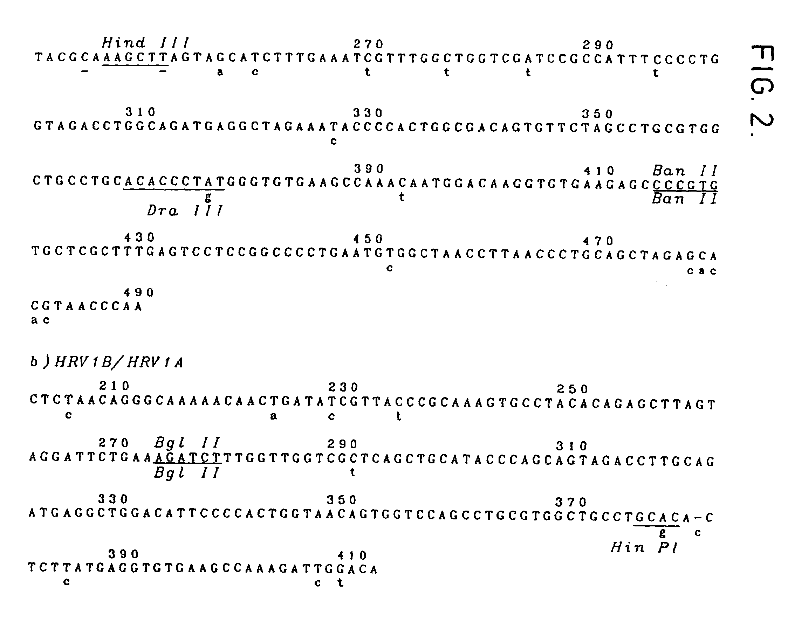

Da keine Sequenzinformationen zum HRV1A, HRV49 und HRV70 zur Verfügung standen, wurde die DNA-Sequenz aus den amplifizierten Fragmenten von HRV1A und HRV49 bestimmt, um zu ermitteln, ob charakteristische Restriktionsstellen präsent sind. Dies Ergebnis korrelliert gut mit dem Grad an Kreuzreaktivität zwischen diesen Paaren (Cooney et al., 1982). Die Unterschiede zwischen den Sequenzen von HRV2 und HRV49 und zwischen denen von HRV1A und HRV1B sind in den Figuren 2a bzw. 2b dargestellt. Insgesamt konnten 15 Basenaustausche und 2 Deletionen zwischen HRV2 und HRV49 innerhalb der 241 sequenzierten Basenpaare beobachtet werden. Bei den 210 bp, die von HRV1A und HRV1B analysiert wurden, konnten 9 Basenaustausche und 1 Insertion beobachtet werden. Diese Veränderungen führten in beiden Fällen zur Bildung oder zum Verlust von einer oder mehr Restriktionsschnittstellen (s. Fig. 2). Mit Hilfe dieses Amplifikationsexperimentes konnte also gezeigt werden, daß für jeden Serotyp charakteristische Schnittstellen ausgewählt werden können.

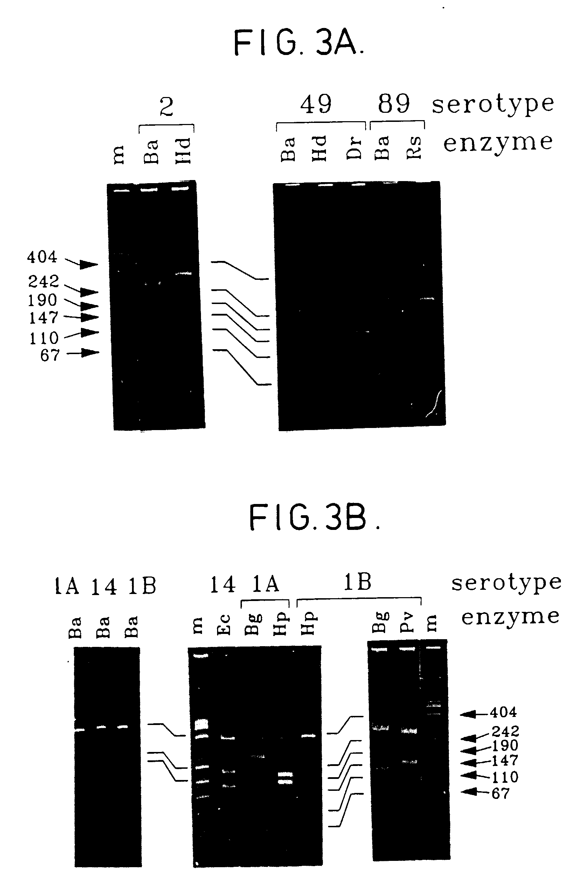

Um die erfindungsgemäße Aufgabe zu lösen, wurden solche Restriktionsenzyme ausgewählt, mit denen die Serotypen zweifelsfrei identifiziert werden können; Tabelle 1 zeigt die ausgewählten Enzyme zusammen mit den Fragmentstellen für das rhinovirale System. Ein Vergleich der DNA-Fragmente zeigte, daß die Fragmente von HRV2, HRV49 und HRV89 für das Enzym BanII eine Schnittstelle aufweisen (Fig. 3a), die Fragmente von HRV1A, HRV1B und HRV14 jedoch nicht (Fig. 3b). Vorzugsweise lassen sich durch die Verwendung dieses Enzyms die Serotypen daher in zwei Gruppen aufteilen und erleichtern damit die Identifizierung der Serotypen: die amplifizierten Fragmente jedes Serotyps wurden mit BanII geschnitten und die Produkte auf Polyacrylamidgelen analysiert. Auch die unterschiedlichen Fragmentlängen, die bei HRV2 und HRV89 erhalten wurden, erlauben die Identifizierung dieser Serotypen wie in Fig. 3a gezeigt ist. Die charakteristische HindIII-Stelle des HRV2 sowie die RsaI-Stelle des HRV89 bestätigten diese Identifizierung. HRV2 konnte durch die Abwesenheit der HindIII-Stelle im HRV49 von diesem unterschieden werden.

Die Serotypen, die keine BanII-Stelle aufweisen (Fig. 3b), wurden folgendermaßen identifiziert: HRV14 wurde über die EcoRI-Stelle identifiziert. HRV1B und HRV1A weisen beide eine BglII-Stelle an derselben Position auf; hier erlaubte die Präsenz einer HinPI-Stelle im HRV1A die eindeutige Unterscheidung der beiden Serotypen.

Erfindungsgemäß ist es daher überraschenderweise möglich, auch unbekannte Viren zu typisieren. So hätten beispielsweise bei Anwesenheit der 6 Serotypen in einem Blindversuch durch Verdau mit BanII und anschließend mit HindIII HRV2, HRV49 und HRV89 eindeutig identifiziert werden können.

Eine Überprüfung der Datenbanken ergab zwar, daß die erfindungsgemäßen Primer ebenfalls zur Amplifikation von RNA sämtlicher Stämme Poliovirus Typ 1 und 2, jedoch nur des Stammes 23127 des Typs 3 (Cameron 1988) geeignet sind. Außerdem ist bekannt, daß Poliovirus aus Nasalmembranen von Patienten sezerniert wird, die das Sabin-Vaccin erhalten haben; RNA der Typen 1 und 2 würde daher bei einem erfindungsgemäßen Typisierungsversuch ebenfalls amplifiziert werden. Berücksichtigt man jedoch den beachtlichen Grad an Unterschieden zwischen Rhinovirus und Poliovirus, so gibt es charakteristische Restriktionsstellen für Poliovirus, mit denen jedwede Mißinterpretation auszuschließen ist. Die Primer können andererseits aber auch vorteilhaft verwendet werden, um die Schnelligkeit des U/C-Wechsel an Position 472 von Poliovirus zu überprüfen, ein Wechsel, von dem gezeigt worden war, daß er für die Virulenz verantworlich ist und daß er bei der Passage der Sabin-Stämme im Menschen auftritt (Cann et al., 1984). Unter stringenten Amplifikationsbedingungen ist eine Amplifikation der Coxsackie-Viren mit diesen Primern nicht zu erwarten.

Die Verfügbarkeit dieser schnellen, erfindungsgemäßen Methode zur Typisierung von Viren wird die Identifizierung beispielsweise der zirkulierenden Rhinovirus-Serotypen innerhalb einer Population ermöglichen und damit epidemiologische Studien erleichtern. Um die Anwesenheit irgendeines Virus zu bestimmen, werden stets nasale Sekretspülungen routinemäßig in HeLa-Zellen eingebracht, was notwendig ist, um ausreichende Mengen für weitere Analysen zu erhalten. Die in den hier beschriebenen Experimenten benötigte Menge an Material für ein Amplifikationsexperiment ist ähnlich der, die man nach einer einmaligen Passage erhält; empfehlenswert ist es aber, mit den nasalen Sekretspülungen direkt zu arbeiten, wie dies durch Gama et al. (1988) beschrieben wurde. Es war sogar möglich, die cDNA von einer RNA, die aus einem einzigen Plaque isoliert worden war, mit den hier verwendeten Primern zu amplifizieren. Kürzlich wurde berichtet, daß Oligonukleotide, die zu Sequenzen aus den 5′-nicht kodierenden Regionen von Rhinoviren komplementär waren, verwendet werden konnten, um Rhinovirus RNA von über 50 Serotypen in nasalen Sekretspülungen nachzuweisen (Bruce et al., 1988). Es ist möglich, daß die hier beschriebenen Primer in Verbindung mit einem ähnlichen Hybridisierungsassay verwendet werden können, um die Empfindlichkeit zu verbessern und um zwischen verschiedenen Serotypen unterscheiden zu können.

Zusammengefaßt bleibt festzuhalten: es konnte gezeigt werden, daß die PCR in Verbindung mit zwei Primern verwendet werden kann, um ein DNA-Fragment von verschiedenen Rhinovirus-Serotypen zu amplifizieren. Durch den Einsatz verschiedener spezifischer Restriktionsenzyme auf diese amplifizierten Fragmente konnte dann zwischen den verschiedenen Serotypen unterschieden werden.
Gegenstand der vorliegenden Erfindung ist eine Methode, mit der man Rhinoviren , die ein Arrangement konservierter Sequenzblocks zwischen verschiedenen Serotypen aufweisen, typisieren kann.

Anfänglich wird es noch nötig sein , die amplifizierte DNA von solchen Rhinoviren die kein bekanntes Restriktionsenzymmuster besitzen, zu sequenzieren und den Serotyp mit Antikörpern zu identifizieren. Auf diese Weise wird es möglich sein, einen Katalog von Restriktionsenzymmustern beispielsweise aller Rhinovirus-Serotypen zu erstellen, ein Katalog, der regelmäßig kontinuierlich auf den neuesten Stand gebracht werden kann und der auf Dauer eine Antikörper-Typisierung überflüssig werden läßt.

Mit Hilfe dieser Kataloge und des erfindungsgemäßen Verfahrens ist es daher möglich, Viren auf schnelle Art zu charakterisieren und zu typisieren.

Die vorliegende Erfindung betrifft daher ein Verfahren zur Charakterisierung von, Rhinoviren, sowie ein Kit zur Durchführung dieses Verfahrens gemäß den Ansprüchen 1-6.

### Legende zu den Figuren

- Figur 1:: Polyacrylamidgelanalyse der PCR-Produkte. Aliquote amplifizierter cDNA, die von einer RNA erhalten wurde, die aus Rohlysat-Präparationen (Serotypen 1B, 2, 49, 70 und 89) oder aus mit Rhinovirus infizierten HeLa-Lysaten (Serotypen 1A und 14) isoliert worden war. Die Serotypen sind am Kopf der Banden angegeben; m: Marker-DNA; c: Kontrolle mit aus nicht-infizierten HeLa-Zellen isolierter RNA. Die Größen (in Basenpaaren) sowohl von drei Markerfragmenten als auch vom amplifizierten Fragment sind angegeben.
- Figur 2:: Verwandtschaft zwischen humanen Rhinovirus-Serotypen in der 5′-nicht-kodierenden Region.
a) HRV2 und HRV49
   Die HRV2 Sequenz von Nukleotid 241 bis 490 ist dargestellt. Unterschiede zwischen dieser und der HRV49 Sequenz (bestimmt aus den Nukleotiden korrespondierend zu 241 bis 482) sind unterhalb angegeben.
b) HRV1A und HRV1B
   Die HRV1B Sequenz von Nukleotid 203 bis 412 ist dargestellt. Unterschiede zwischen dieser und der HRV1A Sequenz (aus der entsprechenden Region bestimmt) sind unterhalb angegeben.
   Ein Strich markiert Deletion eines Nukleotids. Restriktionsstellen, die verwendet wurden, sind angegeben.
- Figur 3:: Polyacrylamidgelanalyse der durch Restriktionsenzymverdau der amplifizierten DNA erhaltenen Fragmente.
A) BanII positive Serotypen
B) BanII negative Serotypen
   Die amplifizierten DNA Fragmente wurden mit den in Tabelle 1 angegebenen Enzymen verdaut. Die Serotypen und Enzyme sind oberhalb der einzelnen Banden angegeben.
   Abkürzungen : Ba: BanII, Bg: BglII; Dr: DraIII; Ec: EcoRI,; Hi: HinPI; Hd: HindIII; Pv: PvuII; Rs: RsaI; m: Marker. Die Größen der Marker sind in Basenpaaren angegeben.

### Materialien und Methoden

### Produktion von Rohlysat-Präparationen der HRV-Stämme

Sämtliche Virus Serotypen wurden von der ATCC erhalten und plaque-gereinigt. HeLa Zellen (Stamm Ohio) wurden in 150 cm² T-Kolben kultiviert und bei einer MOI von ungefähr 1 wie bei Skern et al. (1984) beschrieben mit den HRV-Stämmen infiziert. Die Menge an Virus betrug üblicherweise 10⁹ PFU in 30 ml Medium. Nach zwei Zyklen Frieren/Tauen zur Zellyse wurde das Medium von Zelltrümmern durch niedrigtourige Zentrifugation geklärt. Das Virus wurde mit Polyethylenglykol 6000 (PEG) aus dem Medium konzentriert und in 1 ml Phosphat-gepufferter Salzlösung resuspendiert. Bei Verwendung von infizierten HeLa-Lysaten wurde auf die PEG-Präzipitation verzichtet.

### Reverse Transkription der viralen RNA

Die RNA wurde durch Behandeln von 0,1 - 0,5 ml der viralen, PEG konzentrierten Suspension oder von 0,5 ml nicht-konzentrierter Suspension mit 1% SDS und 10 mM EDTA hergestellt. Nach der Extraktion mit Phenol/Chloroform wurden 2 µg der Carrier tRNA zugegeben und die RNA mit Ethanol präzipitiert. Die cDNA wurde hergestellt, indem die gesamte RNA-Präparation, 10 pmol des Primer 2 und 10 Einheiten Reverse Transkriptase (Super RT, Anglian Biotechnology) in 20 µl Endvolumen entsprechend der Vorschriften des Herstellers eingesetzt wurde. Anfänglich wurde die cDNA durch Extraktion mit Phenol/Chloroform und Ethanol-Präzipitation gereinigt; in späteren Experimenten wurde die cDNA-Mischung direkt für die "Polymerase Chain Reaction" verwendet.

### "Polymerase Chain Reaction"

Die "Polymerase Chain Reaction" (PCR) wurde in einem Gesamtvolumen von 50 µl mit 10 µl der cDNA-Präparation, jeweils 100 pmol Primer 1 und Primer 2, 0,4 mM aller vier dNTPs, 2 Einheiten von Thermus aquaticus DNA Polymerase (Cetus) im Puffer der Firma Cetus Corp., unter Verwendung des von Torgersen et al. (1989) beschriebenen Apparates für 30 Zyklen bei einer Einstellung von 92°C (2 min.), 40°C (3 Min.) und 70°C (3 Min.) durchgeführt. 10 µl der Reaktionsmischung wurden direkt auf einem 6% Polyacrylamid-Gel (Maniatis et al., 1982) analysiert. Restriktionsanalysen wurden an Aliquoten der amplifizierten DNA, unter Verwendung der in Tabelle 1 gezeigten Enzyme durchgeführt; die Produkte wurden auf 6% Polyacrylamid-Gel analysiert.

Zur Sequenzierung wurde die Dideoxy Sequenzierungsmethode nach Sanger verwendet (Sanger et al., 1977). Die nach der PCR erhaltene doppelsträngige DNA wurde von dem Polyacrylamid-Gel elektroeluiert und unter Verwendung der Primer 1 und 2 und der modifizierten T7 Polymerase (Pharmacia) entsprechend der Herstellervorschriften sequenziert. Die Computeranalyse der DNA-Sequenzen wurde unter Verwendung der von Isono modifizierten Staden-Programme durchgeführt (Isono, 1982).

### Ergebnisse

Ein Vergleich der Nukleotidsequenzen von HRV1B (Hughes et al., 1988), HRV2 (Skern et al., 1985) und HRV89 (Duechler et al., 1987) zeigte Identität der Regionen zwischen den Nukleotiden # 161 und 181 bzw. # 531 und 553 (die Numerierung erfolgte gemäß HRV2, Skern et al., 1985). Überraschenderweise weisen die vier Serotypen untereinander signifikante Unterschiede zwischen diesen Regionen identischer Klammersequenzen auf. Die sich hieraus ergebenden charakteristischen Unterschiede im Restriktionsmuster jedes Serotyps, die auch in den von diesen Regionen ausgehenden amplifizierten Fragmenten vorhanden sind, werden erfindungsgemäß verwendet, um die der Erfindung zugrunde liegende Aufgabe zu lösen. Hierzu wurden zwei Primer synthetisiert: Oligonukleotid-Primer 1 (Sequenz # 161 bis 178: CAAGCACTTCTGTTTCCC) und Oligonukleotid-Primer 2 (komplementär zu # 531 bis 544: ACTACTTTGGGTGT). Die CDNA wurde aus viraler RNA hergestellt und unter Verwendung der Primer 1 und 2 wie oben beschrieben amplifiziert.

Fig. 1 zeigt die Polyacrylamidgelanalyse eines Amplifikationsexperiments, bei dem 7 verschiedene HRV-Serotypen verwendet wurden. In allen Fällen wurde ein DNA-Fragment von annähernd 380 bp generiert (das entspricht dem Abstand zwischen den beiden Primern). Damit konnte gezeigt werden, daß die Primer in der Lage sind, auch an die cDNA sowohl des HRV1A, als auch HRV49 und HRV70 zu binden, implizierend, daß die entsprechenden Sequenzen auch in diesen Serotypen präsent sind. Da keine Sequenzinformationen von HRV1A, HRV49 und HRV70 zur Verfügung standen, wurde die DNA-Sequenz aus den amplifizierten Fragmenten von HRV1A und HRV49 bestimmt, um zu ermitteln, ob charakteristische Restriktionsstellen präsent sind. 241 bp der Sequenz für HRV49 und 210 bp für HRV1A wurden erhalten. Eine Computer-Analyse ergab, daß die Sequenz des HRV49 der des HRV2 und die Sequenz des HRV1A der des HRV1B eng verwandt ist (um die Genauigkeit der HRV49 Sequenz zu verbessern, wurden die Sequenzierungsreaktionen unter Verwendung der Primer 1 und 2 an einem Plasmid durchgeführt, das die HRV2 5′-nicht kodierende Region enthielt, so daß die Unterschiede eindeutig feststellbar waren). Die Unterschiede zwischen den Sequenzen von HRV2 und HRV49 und zwischen denen von HRV1A und HRV1B sind in den Figuren 2a bzw. 2b dargestellt. Insgesamt konnten 15 Basenaustausche und 2 Deletionen zwischen HRV2 und HRV49 innerhalb der 241 sequenzierten Basenpaare beobachtet werden. Bei den 210 bp, die von HRV1A und HRV1B analysiert wurden, konnten 9 Basenaustausche und 1 Insertion beobachtet werden. Diese Veränderungen führten in beiden Fällen zur Bildung oder zum Verlust einer Restriktionsschnittstelle (wie in Fig. 2 dargestellt, ging bezogen auf HRV2 im HRV49 eine HindIII-Stelle verloren und eine DraIII-Stelle wurde geschaffen). Mit Hilfe dieses Amplifikationsexperimentes konnte also gezeigt werden, daß für jeden Serotyp charakteristische Schnittstellen ausgewählt werden können.

Um die erfindungsgemäße Aufgabe zu lösen, wurden solche Restriktionsenzyme ausgewählt, mit denen die Serotypen zweifelsfrei identifiziert werden können; Tabelle 1 zeigt die ausgewählten Enzyme zusammen mit den Fragmentstellen. Für HRV1A und HRV49 wurde unterstellt, daß in dem unbekannten Bereich keine weiteren Stellen für die entsprechenden Enzyme vorliegen. Ein Vergleich der DNA-Fragmente zeigte, daß die Fragmente von HRV2, HRV49 und HRV89 für das Enzym BanII eine Schnittstelle aufweisen (Fig. 3a), die Fragmente von HRV1A, HRV1B und HRV14 jedoch nicht (Fig. 3b). Vorzugsweise lassen sich durch die Verwendung dieses Enzyms die Serotypen in zwei Gruppen aufteilen: Um die Identifizierung der Serotypen zu vereinfachen, wurden die amplifizierten Fragmente jedes Serotyps mit BanII geschnitten und die Produkte auf Polyacrylamidgelen analysiert. Auch die unterschiedlichen Fragmentlängen, die bei HRV2 und HRV89 erhalten wurden, erlauben die Identifizierung dieser Serotypen wie in Fig. 3a gezeigt ist. Die charakteristische HindIII-Stelle des HRV2 sowie die Rsal-Stelle des HRV89 bestätigten die Identifizierung (das 96 bp RsaI-Fragment war zu schwach, um auf diesem Gel gesehen zu werden). HRV2 und HRV49 konnten durch die Abwesenheit der HindIII-Stelle im HRV49 unterschieden werden.

Die Serotypen, die keine BanII-Stelle aufweisen (Fig. 3b), wurden folgendermaßen identifiziert: HRV14 wurde über die EcoRI-Stelle identifiziert (der Verdau erfolgte teilweise). HRV1B und HRV1A haben beide eine BglII-Stelle an derselben Position; hier erlaubte die Präsenz einer HinPI-Stelle im HRV1A die eindeutige Unterscheidung der beiden Serotypen. Da nur zwei Fragmente mit HinPI erhalten wurden, muß die in Fig. 2 markierte Stelle tatsächlich die einzige in dem amplifizierten Fragment sein.

### Literaturverzeichnis

Bruce, C.B., Al-Nakib, W., Tyrell, D.A.J. and Almond, J.W. (1988). Synthetic oligonucleotides as diagnostic probes. Lancet, 8601, 53.

Callahan, P.L., Mizutani, S., and Colonno, R.J. (1985). Molecular cloning and complete sequence determination of RNA sequence of human rhinovirus type 14. Proc. Natl. Acad. Sci. U.S.A., 82 732-736.

Cameron, G.N. (1988). The EMBL data library. Nucleic Acids Res., 16, 1865-1867.

Cann, A., Stanway, G., Hughes, P.J., Evans, D.M.A., Schild, C.C. and Almond, J.W. (1984). Reversion to neurovirulence of the live-attenuated Sabin type 3 oral poliovirus vaccine. Nucleic Acids Res., 12, 7787-7792.

Cooney, M.K., Fox, J.P. and Kenney, G.E. (1982). Antigenic groupings of 90 rhinovirus serotypes. Infect. Imm., 37, 642-647.

Duechler, M., Skern, T., Sommergrüber, W., Neubauer, Ch., Gruendler, P., Fogy, I., Blaas, D. and Kuechler, E. (1987). Evolutionary relationships within the human rhinovirus genus; comparison of serotypes 89, 2 and 14. Proc. Natl. Acad. Sci. U.S.A., 84, 1605-2609.

Gama, R.E., Hughes, P.J., Bruce, C.B. and Stanway, G. (1988). Polymerase chain reaction amplification of rhinovirus nucleic acids from clinical material. Nucleic Acids Res., 16, 9346.

Hamparian, V.V., Colonno, R.J., Cooney, M.K., Dick, E.C., Gwaltney, J.M., Jr. Hughes, J.H., Jordan, W.S., Kapikian, A.Z., Mogabgab, W.J., Monto, A., Philips, C.A., Rückert, R.R., Schieble, J.H., Stott, E.J. and Tyrell, D.A.J. (1987). A collaborative report: Rhinoviruses - Extension of the numbering system from 89 to 100. Virology, 159, 191-192.

Hughes, P.J., North, C., Jellis, C.H., Minor, P.D. and Stanway, G. (1988). The nucleotide sequence of human rhinovirus 1B: molecular relationships within the rhinovirus genus. J. Gen. Virol., 69, 49-58.

Isono, K. (1982). Computer programs to analyse DNA and amino acid sequence data. Nucleic. Acids. Res., 10, 85-89.

Kellner, G., Popow-Kraupp, T., Kundi, M., Binder, C., Mallner H. and Kunz, C. (1988). Contribution of rhinoviruses respiratory infections in childhood: a prospective study in a mainly hospitalized infant population. J. Med. Virol., 25, 455-469.

Maniatis, T., Fritsch, F. and Sambrook, J. (1982). Molecular cloning, a laboratory manual. Cold Spring Harbour Laboratory, Cold Spring Harbour, New York.

Rivera, V.M., Welsh, J.D. and Maizel, J.V. (1988). Comparative sequence analysis of the 5′non-coding region of enteroviruses and rhinoviruses. Virology 165, 42-50.

Saiki, R.K., Gelfand, D.H., Stoffel, S., Scharf, St.J., Higuchi, R., Horn, G.T., Mullis, K.B. and Erliche, H.A. (1988). Primer directed enzymatic amplification of DNA with a thermostable DNA polymerase. Science 239, 487-491.

Sanger, F., Nickley, S. and Coulson, A.R. (1977). DNA sequencing with chain-terminating inhibitors. Proc. Natl. Acad. Sci. U.S.A., 74, 5463-5467.

Skern, T., Sommergruber, W., Blaas, D., Pieler, Ch. and Kuechler, D. (1984). Relationship of human rhinovirus strain 2 and poliovirus as indicated by comparison of the polymerase gene regions. Virology 136, 125-132.

Skern, T., Sommergruber, W., Blaas, D., Gruendler, P., Fraundorfer, F., Pieler, C., Fogy, I. and Kuechler, B. (1985). Human Rhinovirus 2: complete nucleotide sequence and proteolytic processing signals in the capsid protein region. Nucleic Acids Res., 13, 2111-2126.

Stanway, G., Hughes, P.J., Mountford, R.C., Minor, P.D. and Almond, J.W. (1984). The complete nucleotide sequence of a common cold virus: human rhinovirus 14. Nucl. Acids. Res., 12, 7859-7875.

Stott, E.J. and Killington, R.A. (1972). Rhinoviruses. Ann. Rev. Microbiol., 26, 503-525.

Torgersen, H., Blaas, D. and Skern, T. (1989). Low cost apparatus for primer-directed DNA amplification. Anal. Biochem., 176, 33-35.

R.E. Gama, P.R. Horsnell, P.J. Hughes, C. North, C.B. Bruce, W. Al-Nakib and G. Stanway (1989). Amplification ofRhinovirus Specific Nucleic Acids From Clinical Samples Using the Polymerase Chain Reaction. Journal of Medical Virology 28:73-77.

F. Morinet, J.-D. Tratschin, Y. Perol and G. Siegl (1986). Comparison of 17 Isolates ofthe Human Parvovirus B 19 by Restriction Enzyme Analysis. Archives of Virology 90, 165-172.

### Europäische Patentanmeldung Nr. 0 229701

Sninsky, J.J., Kwok, S.L., Mack, D.H., Detection ofviruses by amplification and hybridization. Cetus Corporation (US).

### Europäische Patentanmeldung Nr. 0 309969

Livak, K.J., Brenner, S., Method ofgene mapping. E.I. Du Pont de Nemours and Company (US).

### Internationale Patentanmeldung WO 87/02065

Hall, B., Gordon D., Determination ofIdentity between two Organisms. Biotechnica Limited (GB).

**Tab. 1:**

| Bei der Identifizierung der Rhinovirus Serotypen verwendete Restriktionsstellen. Die Zahlen geben Basenpaare an. Ein Strich bedeutet, daß keine Restriktionsstelle für das spezielle Enzym vorhanden ist. NU gibt an, daß zwar eine Restriktionsstelle vorhanden ist, doch nicht verwendet wurde. | | | | | | |
|---|---|---|---|---|---|---|
| **Serotyp** | | | | | | |
| | HRV1A | HRV1B | HRV2 | HRV14 | HRV49 | HRV89 |
| Ban II | - | - | 255 | - | 255 | 298 |
| Bgl II | 281 | 281 | - | - | - | - |
| | 106 | 106 | | | | |
| Dra III | - | - | - | - | 212 | - |
| | | | | | 172 | |
| Eco RI | - | - | - | 215 | - | - |
| | | | | 165 | | |
| Hin PI | 208 | - | NU | - | NU | - |
| | 179 | | | | | |
| Hind III | - | - | 297 | - | - | - |
| | | | 87 | | | |
| Pvu II | NU | 260 | - | | - | - |
| | | 127 | | | | |
| Rsal | NU | - | - | NU | - | 294 |
| | | | | | | 94 |

## Patentansprüche

1. Verfahren zur Typisierung humaner Rhinoviren, dadurch gekennzeichnet, daß
a) RNA eines Rhinovirus in cDNA überführt wird,
b) die cDNA mittels PCR amplifiziert wird, dadurch gekennzeichnet, daß ein Primer eine Sequenz besitzt die an die Sequenz GGG AAA CAG AAG TGC TTG bindet und ein weiterer Primer eine Sequenz besitzt die an die Sequenz ACT ACT TTG GGT GT bindet,
c) die amplifizierte DNA mit mindestens einem Restriktionsenzym ausgewählt aus der Gruppe BanII, HindIII, RsaI, EcoRI, BglII, PvuII, DraIII oder HinPI geschnitten wird und
d) das erhaltene Restriktionsmuster mit dem Restriktionsmuster bekannter Viren verglichen wird.

2. Verfahren nach Anspruch 1, dadurch gekennzeichnet, daß ein Primer die Sequenz CAA GCA CTT CTG TTT CCC und ein weiterer Primer die Sequenz ACA CCC AAA GTA GT besitzt.

3. Verfahren nach Anspruch 1 oder 2, dadurch gekennzeichnet, daß der humane Rhinovirus aus einer Gruppe ausgewählt ist die HRV1A, HVR1B, HRV2, HRV14, HRV49 und HRV89 enthält.

4. Kit zur Durchführung des Verfahrens zur Typisierung humaner Rhinoviren gemäß Anspruch 1 dadurch gekennzeichnet, daß er
a) einen Primer enthält der an die Sequenz GGG AAA CAG AAG TGC TTG bindet und
b) einen weiteren Primer enthält der an die Sequenz ACT ACT TTG GGT GT bindet und
c) mindestens ein Restriktionsenzym ausgewählt aus der Gruppe BanII, HindIII, RsaI, EcoRI, BglII, PvuII, Dram oder HinPI enthält.

5. Kit nach Anspruch 4, dadurch gekennzeichnet, daß er einen Primer der Sequenz CAA GCA CTT CTG TTT CCC und einen weiteren Primer der Sequenz ACA CCC AAA GTA GT enthält.

6. Kit nach einem der Ansprüche 4 oder 5, dadurch gekennzeichnet, daß er eine reverse Transkriptase und eine DNA Polymerase enthält.

## Claims

1. Process for typing human rhinoviruses,
characterised in that
a) RNA of a rhinovirus is converted into cDNA,
b) the cDNA is amplified by PCR, characterised in that a primer has a sequence which binds to the sequence GGG AAA CAG AAG TGC TTG and another primer has a sequence which binds to the sequence ACT ACT TTG GGT GT,
c) the amplified DNA is cut with at least one restriction enzyme selected from the group comprising BanII, HindIII, RsaI, EcoRI, BglII, PvuII, DraIII or HinPI and
d) the restriction pattern obtained is compared with the restriction pattern of known viruses.

2. Process according to claim 1, characterised in that one primer has the sequence CAA GCA CTT CTG TTT CCC and another primer has the sequence ACA CCC AAA GTA GT.

3. Process according to claim 1 or 2, characterised in that the human rhinovirus is selected from a group which contains HRV1A, HVR1B, HRV2, HRV14, HRV49 and HRV89.

4. Kit for carrying out the process for typing human rhinoviruses according to claim 1, characterised in that it contains
a) a primer which binds to the sequence GGG AAA CAG AAG TGC TTG and
b) another primer which binds to the sequence ACT ACT TTG GGT GT and
c) at least one restriction enzyme selected from the group comprising BanII, HindIII, RsaI, EcoRI, BglII, PvuII, DraIII or HinPI.

5. Kit according to claim 4, characterised in that it contains one primer of the sequence CAA GCA CTT CTG TTT CCC and another primer of the sequence ACA CCC AAA GTA GT.

6. Kit according to one of claims 4 or 5,
characterised in that it contains a reverse transcriptase and a DNA polymerase.

## Revendications

1. Procédé de caractérisation de rhinovirus humains,
caractérisé en ce que
a) l'ARN d'un rhinovirus est converti en ADNc,
b) l'ADNc est amplifié par ACP, caractérisé en ce qu'une amorce possède une séquence qui se lie à la séquence GGG AAA CAG AAG TGC TTG et une autre amorce possède une séquence qui se lie à la séquence ACT ACT TTG GGT GT,
c) l'ADN amplifié est coupé avec au moins une enzyme de restriction choisie dans le groupe BanII, HindIII, RsaI, EcoRI, BglII, PvuII, DraIII ou HinPI et
d) le motif de restriction obtenu est comparé au motif de restriction de virus connus.

2. Procédé selon la revendication 1, caractérisé en ce qu'une amorce possède la séquence CAA GCA CTT CTG TTT CCC et une autre amorce possède la séquence ACA CCC AAA GTA GT.

3. Procédé selon la revendication 1 ou 2, caractérisé en ce que le rhinovirus humain est choisi dans un groupe qui contient HRV1A, HRV1B, HRV2, HRV14, HRV49 et HRV89.

4. Trousse pour la mise en oeuvre du procédé de caractérisation de rhinovirus humains selon la revendication 1, caractérisée en ce qu'elle contient
a) une amorce qui se lie à la séquence GGG AAA CAG AAG TGC TTG et
b) une autre amorce qui se lie à la séquence ACT ACT TTG GGT GT et
c) au moins une enzyme de restriction choisie dans le groupe BanII, HindIII, RsaI, EcoRI, BglII, PvuII, DraIII ou HinPI.

5. Trousse selon la revendication 4, caractérisée en ce qu'elle contient une amorce de séquence CAA GCA CTT CTG TTT CCC et une autre amorce de séquence ACA CCC AAA GTA GT.

6. Trousse selon l'une des revendications 4 ou 5, caractérisée en ce qu'elle contient une transcriptase inverse et une ADN polymérase.
